# EUROPEAN PATENT APPLICATION

(11) **EP 4 797 281 A1**
(43) Date of publication of application: **26.08.2026**
(21) Application number: 25874031.5
(22) Date of filing: 23.09.2025
(51) Int. Cl.: G16H 50/70, G16H 50/50, G16H 30/40, G16H 30/20, G06V 10/764, G06V 10/82, G06T 7/11, G06T 3/40, G06N 3/096, G06N 3/0464

(54) **SYSTEM AND METHOD FOR DIAGNOSING OSTEOPENIA BY USING X-RAY IMAGES**

(30) Priority: 26.09.2024 KR 20240130751; 22.09.2025 KR 20250136534
(71) Applicant: Promedius Inc., Seoul 05510 (KR)
(72) Inventor: KIM, Minje, Seoul 08054 (KR); PARK, Junhyeok, Seoul 06932 (KR); PARK, Saerom, Seoul 05730 (KR)
(74) Representative: Lavoix
(86) International application number: PCT/KR2025/014904
(87) International publication number: WO 2026/071677

(57) **Abstract**

A system for diagnosing osteopenia includes: an artificial intelligence model training device that includes a first artificial intelligence model training device training a first artificial intelligence model that classifies an input image as osteoporosis or non-osteoporosis and a second artificial intelligence model training device training a second artificial intelligence model that classifies a non-osteoporosis image as normal condition or the osteopenia; and an osteopenia prediction device that initially classifies an input image for diagnosis as the osteoporosis or the non-osteoporosis based on the first artificial intelligence model and classifies the initially classified non-osteoporosis image as the normal condition or the osteopenia based on the second artificial intelligence model.

## Description

### [Technical Field]

The present disclosure relates to a system and a method for diagnosing osteopenia using an X-ray image, and more particularly, to a system and a method for diagnosing osteopenia using an entire image and a segmented image of a chest X-ray.

### [Background Art]

Republic of Korea entered an aged society in 2018 where a proportion of elderly people aged 65 or older is 14% of the total population, and is expected to enter an ultra-aged society with more than 20% in 2025. Accordingly, the number of patients with bone disease is expected to increase rapidly, and medical and socioeconomic costs caused by the bone disease are expected to increase rapidly.

A representative bone disease may include osteoporosis and osteopenia. The osteoporosis refers to a state in which a bone density and a bone strength are lowered so that fracture is more likely to occur, and the osteopenia is a prestage of the osteoporosis and refers to a state in which a bone density is lower than a normal bone density. The osteoporosis and the osteopenia do not have a specific symptom but a possibility of each of a secondary fracture and a complication increases in the osteoporosis and the osteopenia if a fracture occurs so that it is necessary to prevent a risk of the osteoporosis or the osteopenia through a screening test for reduction of the bone density. The osteoporosis and the osteopenia are diagnosed through a bone density test such as dualenergy X-ray absorptiometry (DEXA), and a state of a bone is classified as a normal condition, osteopenia, or osteoporosis based on a bone index (e.g.,T-score) that is a value measured after the bone density test.

As an image analysis technology using an artificial intelligence model has recently developed dramatically, the artificial intelligence model is also being utilized in diagnosis of the bone disease. U.S. Patent No. 12,033,318 describes a technology for estimating a bone density from an X-ray image using the artificial intelligence model. Specifically, according to U.S. Patent No. 12,033,318, training for the artificial intelligence model may be performed using a human skeletal image as training data and bone mineral density (BMD) measured from the DEXAs supervised data for supervised training, and the bone density or a bone index (e.g., a T-score or the like) with continuity may be directly predicted from a chest X-ray image through the artificial intelligence model in which the training is completed so that a state of a bone or presence or absence of the bone disease is determined.

A technology described in U.S. Patent No. 12,033,318 has an advantage of providing detailed diagnosis information such as the state of the bone through a continuous prediction value of the bone density or the bone index. However, in order to train a highly accurate artificial intelligence model, in the technology described in U.S. Patent No. 12,033,318, a large amount of medical images from various hospitals (or cohort groups) and DEXA extraction label information paired therewith (e.g., the bone density or bone index information such as the T-score) are required.

Instead of using the bone density as a training label, the artificial intelligence model has recently been trained by constructing a training dataset with an entire X-ray image of each of a normal person and an osteoporosis patient and a categorical label (normal condition/osteoporosis) therefor, and a technology estimating osteoporosis from the X-ray image using the artificial intelligence model in which the training is completed is being developed. However, a technology diagnosing osteopenia from the X-ray image using the artificial intelligence model has not yet been developed.

In order to train the artificial intelligence model using data from a normal person and an osteopenia patient and data (imaging data) from the osteopenia patient as training data, a large amount of training data is required. However, there is a problem in which it is not easy to secure a large amount of medical data necessary for training the artificial intelligence model and securing sufficient data incurs a high cost.

### [Disclosure]

### [Technical Problem]

A problem to be solved by the present disclosure is intended to provide a system and a method for diagnosing osteopenia that additionally classifies the osteopenia through a second artificial intelligence model for an X-ray image initially classified as non-osteoporosis through a first artificial intelligence model.

Another problem to be solved by the present disclosure is intended to provide a system and a method for diagnosing osteopenia capable of accurately classifying a state of a bone as osteoporosis or non-osteoporosis even when an image is incomplete by synthesizing output results of models trained from a plurality of extracted images using an ensemble algorithm to infer bone-related information.

### [Technical Solution]

A system for diagnosing osteopenia according to an embodiment of the present disclosure includes: an artificial intelligence model training device that includes a first artificial intelligence model training device training a first artificial intelligence model that classifies an input image as osteoporosis or non-osteoporosis and a second artificial intelligence model training device training a second artificial intelligence model that classifies a non-osteoporosis image as normal condition or the osteopenia; and an osteopenia prediction device that initially classifies an input image for diagnosis as the osteoporosis or the non-osteoporosis based on the first artificial intelligence model and classifies the initially classified non-osteoporosis image as the normal condition or the osteopenia based on the second artificial intelligence model.

The input image may be a chest X-ray image.

The osteopenia prediction device may include: a non-osteoporosis classification device that classifies the input image for diagnosis as the osteoporosis or the non-osteoporosis based on the first artificial intelligence model; and an osteopenia classification device that classifies the non-osteoporosis image classified by the non-osteoporosis classification device as the osteopenia or the normal condition based on the second artificial intelligence model.

The first artificial intelligence model may include each of a plurality of classification models that trains an entire image or one or more segmented images obtained by segmenting the entire image into anatomical regions; and the first artificial intelligence model training device may train a corresponding classification model among the plurality of classification models based on a training label corresponding to the entire image or the segmented image.

The second artificial intelligence model training device may train the second artificial intelligence model based on an entire image of a normal person or a patient with the osteopenia and a training label corresponding thereto.

The second artificial intelligence model training device may include: an image preprocessing module that performs grayscale truncation preprocessing on a training image that is the entire image of the normal person or the patient with the osteopenia; a training dataset generation module that generates a training dataset based on the training image preprocessed in the image preprocessing module and a training label of the normal condition or the osteopenia related to the training image; and a second artificial intelligence model training module that trains the second artificial intelligence model using the training dataset generated by the training dataset generation module.

The second artificial intelligence model may be a knowledge distillation model.

The second artificial intelligence model training module may include: an augmentation module that performs first augmentation and second augmentation stronger than the first augmentation on the training image preprocessed in the image preprocessing module; a teacher model module that receives the training image on which the first augmentation is performed to output a first logit value regarding a class for the osteopenia or the normal condition; a student model module that receives the training image on which the second augmentation is performed to output a second logit value regarding the class for the osteopenia or the normal condition; a temperature scaling module that applies temperature scaling to the first logit value and the second logit value respectively output from the teacher model module and the student model module to respectively output a first correction logit value and a second correction logit value; a probability value conversion module that performs probability conversion on the first correction logit value and the second correction logit value respectively output from the temperature scaling module to respectively output a first probability value and a second probability value and performs probability conversion on the second logit value output from the student model module to output a third probability value; a loss function calculation module that calculates a loss function based on the first probability value, the second probability value, and the third probability value output from the probability value conversion module; and a parameter update module that updates a parameter of the student model module based on the loss function output from the loss function calculation module.

The loss function calculation module may calculate a cross entropy loss function based on the third probability value output from the probability value conversion module, and may calculate a Kullback-Leibler divergence loss function based on the first probability value and the second probability value output from the probability value conversion module.

The parameter update module may sum the cross entropy loss function calculated in the loss function calculation module and the Kullback-Leibler divergence loss function calculated in the loss function calculation module and then may perform backpropagation through an optimizer to update the parameter of the student model module.

The parameter update module may calculate an exponential moving average value for a parameter of the teacher model module and the parameter of the student model module, and may update the student model module.

A system for diagnosing osteopenia according to another embodiment of the present disclosure includes: an artificial intelligence model training device that includes a first artificial intelligence model training device training a first artificial intelligence model that classifies an input image as osteoporosis or non-osteoporosis and a second artificial intelligence model training device training a second artificial intelligence model that classifies a non-osteoporosis image as normal condition, the osteopenia, or the osteoporosis; and an osteopenia prediction device that initially classifies an input image for diagnosis as the osteoporosis or the non-osteoporosis based on the first artificial intelligence model and classifies the initially classified non-osteoporosis image as the normal condition, the osteopenia, or the osteoporosis based on the second artificial intelligence model.

The osteopenia prediction device may include: a non-osteoporosis classification device that classifies the input image for diagnosis as the osteoporosis or the non-osteoporosis based on the first artificial intelligence model; and an osteopenia classification device that classifies the non-osteoporosis image classified by the non-osteoporosis classification device as the normal condition, the osteopenia, or the osteoporosis based on the second artificial intelligence model.

The second artificial intelligence model training device may include: an image preprocessing module that performs grayscale truncation preprocessing on a training image that is an entire image of a normal person, a patient with the osteopenia, or a patient with the osteoporosis; a training dataset generation module that generates a training dataset based on the training image preprocessed in the image preprocessing module and a training label of the normal condition, the osteopenia, or the osteoporosis related to the training image; and a second artificial intelligence model training module that trains the second artificial intelligence model using the training dataset generated by the training dataset generation module.

The first artificial intelligence model training device may include: a first image segmentation module that receives a source training image that is the entire image of the normal person or the patient with the osteoporosis, segments the source training image into images respectively corresponding to anatomical regions, receives a target training image that is the entire image of the normal person, the patient with the osteopenia, or the patient with the osteoporosis, and segments the target training image into the images respectively corresponding to the anatomical regions; a training dataset generation module that generates a plurality of source training datasets based on the source training image and a plurality of segmentation source images segmented from the source training image and generates a plurality of target training datasets based on the target training image and a plurality of segmentation target images segmented from the target training image, wherein each of the plurality of source training datasets includes a source training label labeled the normal condition or the osteoporosis with the source training image or the plurality of segmentation source images and each of the plurality of target training datasets includes a target training label labeled the non-osteoporosis or the osteoporosis with the target training image or the plurality of segmentation target images; a plurality of source classification model training modules that perform primary training on a corresponding source classification model based on the plurality of source training datasets; and a plurality of target classification model training modules that perform secondary training on a corresponding target classification model based on the plurality of target training datasets.

The first artificial intelligence model training device may further include a transfer learning module that performs transfer learning for a corresponding target classification model training module based on a hidden layer parameter that is a training result of the source classification model training module.

The first image segmentation module may crop and may segment the entire image into the images respectively corresponding to the anatomical regions; and the training image generated by the training dataset generation module may include one or more segmented images among an entire chest image, right clavicle-scapula, left clavicle-scapula, a cervical vertebra, and thoracic vertebra/lumbar vertebra.

The non-osteoporosis classification device may include: a second image segmentation module that segments the input image for diagnosis into the anatomical regions to generate a plurality of segmented images; a bone disease classification inference module that infers a bone disease classification result for each of the input image for diagnosis and the plurality of segmented images segmented by the second image segmentation module based on a corresponding classification model among the plurality of target classification models; and a combination module that combines a plurality of bone disease classification results inferred by the bone disease classification inference module using an ensemble algorithm to diagnose whether the input image for diagnosis is an osteoporosis image or the non-osteoporosis image.

An artificial intelligence model training device according to an embodiment of the present disclosure includes: a first artificial intelligence model training device that trains a first artificial intelligence model classifying an input image as osteoporosis or non-osteoporosis; and a second artificial intelligence model training device that trains a second artificial intelligence model classifying a non-osteoporosis image as normal condition or the osteopenia.

The first artificial intelligence model includes each of a plurality of classification models that trains an entire image or one or more segmented images obtained by segmenting the entire image into anatomical regions.

The first artificial intelligence model training device trains a corresponding classification model among the plurality of classification models based on a training label corresponding to the entire image or the segmented image.

The second artificial intelligence model training device may train the second artificial intelligence model based on an entire image of a normal person or a patient with the osteopenia and a training label corresponding thereto.

The second artificial intelligence model training device may include: an image preprocessing module that performs grayscale truncation preprocessing on a training image that is the entire image of the normal person or the patient with the osteopenia; a training dataset generation module that generates a training dataset based on the training image preprocessed in the image preprocessing module and a training label of the normal condition or the osteopenia related to the training image; and a second artificial intelligence model training module that trains the second artificial intelligence model using the training dataset generated by the training dataset generation module.

The second artificial intelligence model may be a knowledge distillation model.

The second artificial intelligence model training device may train the second artificial intelligence model to classify the non-osteoporosis as the normal condition, the osteopenia, or the osteoporosis.

A method for diagnosing osteopenia from a input image for diagnosis according to an embodiment of the present disclosure includes: training a first artificial intelligence model that classifies an input image as osteoporosis or non-osteoporosis; training a second artificial intelligence model that classifies an input non-osteoporosis image as normal condition or the osteopenia; initially classifying the input image for diagnosis as the osteoporosis or the non-osteoporosis based on the first artificial intelligence model; and classifying the initially classified non-osteoporosis image as the normal condition or the osteopenia based on the second artificial intelligence model.

The first artificial intelligence model may include each of a plurality of classification models that trains an entire image or one or more segmented images obtained by segmenting the entire image into anatomical regions.

The training of the first artificial intelligence model may train a corresponding classification model among the plurality of classification models based on a training label corresponding to the entire image or the segmented image.

The training of the second artificial intelligence model may include: performing grayscale truncation preprocessing on a training image that is an entire image of a normal person or a patient with the osteopenia; generating a training dataset based on the preprocessed training image and a training label of the normal condition or the osteopenia related to the training image; and training the second artificial intelligence model using the training dataset generated by the training dataset generation module.

The training of the second artificial intelligence model may include: performing first augmentation and second augmentation stronger than the first augmentation on the preprocessed training image; receiving, by a teacher model module, the training image on which the first augmentation is performed to output a first logit value regarding a class for the osteopenia or the normal condition; receiving, by a student model module, the training image on which the second augmentation is performed to output a second logit value regarding the class for the osteopenia or the normal condition; applying temperature scaling to the first logit value and the second logit value respectively output from the teacher model module and the student model module to respectively output a first correction logit value and a second correction logit value; performing probability conversion on the first correction logit value and the second correction logit value respectively to output a first probability value and a second probability value; performing probability conversion on the second logit value output from the student model module to output a third probability value; calculating a loss function based on the first probability value, the second probability value, and the third probability value; and updating a parameter of the student model module based on the calculated loss function.

The calculating of the loss function may include: calculating a cross entropy loss function based on the third probability value; and calculating a Kullback-Leibler divergence loss function based on the first probability value and the second probability value.

The updating of the parameter may include summing the calculated cross entropy loss function and the calculated Kullback-Leibler divergence loss function and then performing backpropagation through an optimizer.

### [Advantageous Effects]

Before embodiments infer osteopenia using a second artificial intelligence model specialized for classification of the osteopenia, the embodiments may first classify a state of a bone as osteoporosis, and may infer the osteopenia or normal condition from an image classified as non-osteoporosis excluding the osteoporosis so that a decline in classification performance of the osteopenia due to confusion of classification between the osteoporosis and the osteopenia is prevented.

The embodiments may synthesize an output result of a model trained from each of a plurality of extracted images using an ensemble algorithm to infer bone-related information so that they increase prediction accuracy and provide an accurate analysis result even when an image is incomplete.

### [Brief Description of the Drawings]

FIG. 1 is a view showing a configuration of a medical image system according to an embodiment of the present disclosure.
FIG. 2 is a view showing a computing device implementing a bone disease diagnosis system according to an embodiment of the present disclosure.
FIG. 3 is a view showing a bone disease diagnosis system according to an embodiment of the present disclosure.
FIG. 4 is a view showing an artificial intelligence model training device and an osteopenia prediction device according to a first embodiment of the present disclosure.
FIG. 5 is a view showing a first artificial intelligence model training device and a non-osteoporosis classification device according to the first embodiment of the present disclosure.
FIG. 6 is a view showing the first artificial intelligence model training device according to the first embodiment of the present disclosure.
FIG. 7 is a view showing an example of a segmented chest X-ray (CXR) image according to the first embodiment of the present disclosure.
FIG. 8 is a view showing an example of transfer learning according to the first embodiment of the present disclosure.
FIG. 9 is a view showing a training method of the first artificial intelligence model training device according to the first embodiment of the present disclosure.
FIG. 10 is a view showing the non-osteoporosis classification device according to the first embodiment of the present disclosure.
FIG. 11 is a view showing a combination process performed in a combination module according to the first embodiment of the present disclosure.
FIG. 12 is a view showing a second artificial intelligence model training device according to the first embodiment of the present disclosure.
FIG. 13 is a view showing a second artificial intelligence model training module according to the first embodiment of the present disclosure.
FIG. 14 is a view showing a second artificial intelligence model training method according to the first embodiment of the present disclosure.
FIG. 15 is a view showing an osteopenia classification device according to the first embodiment of the present disclosure.
FIG. 16 is a view showing an artificial intelligence model training device and an osteopenia prediction device according to a second embodiment of the present disclosure.

### [Mode for Invention]

An embodiment of the present disclosure will be described in detail hereinafter with reference to the accompanying drawings so that those skilled in the art could easily implement the embodiment. The present disclosure may be modified in various ways, all without departing from the spirit or scope of the present disclosure. In order to clearly describe the present disclosure, parts or modules that are irrelevant to the description are omitted in the drawings, and similar constituent elements throughout the specification are denoted by similar reference numerals.

Unless explicitly stated to the contrary, the word "comprise" and variations such as "comprises" and "comprising" in the specification should be understood to imply the inclusion of stated elements but not the exclusion of any other elements.

Terms such as a "unit", "portion", "er/or", a "module", and the like described in the specification may mean a unit for processing at least one function or operation, and may be implemented by hardware, software, or a combination of hardware and software.

In the present specification, "transmission or provision" may include not only direct transmission or provision but also indirect transmission or provision through another device or using an indirect route.

In the present specification, an expression described in singular may be interpreted as singular or plural unless an explicit expression such as "one" or "single" is used.

If it is determined that a detailed description of a related known technology obscures a gist of the present disclosure in describing the embodiment of the present disclosure, a detailed description thereof will be omitted.

Hereinafter, the embodiment according to the present disclosure will be described in detail with reference to the accompanying drawings. In the drawings, the same reference numeral is used to refer to the same or similar element.

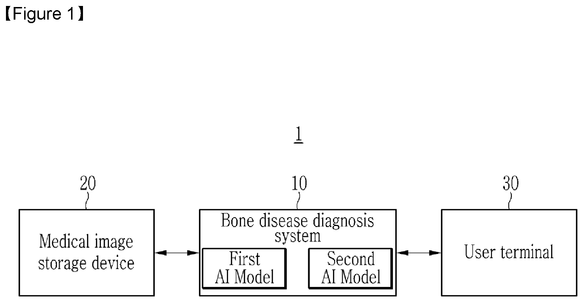

FIG. 1 is a view showing a configuration of a medical image system according to an embodiment of the present disclosure.

Referring to FIG. 1, the medical image system may include at least one user terminal 30, a medical image storage device 20, and a bone disease diagnosis system 10.

The user terminal 30 may include hardware and software that install a program executed by a processor and provide a computing environment and a network environment for performing an operation according to an embodiment of the present disclosure. The user terminal 30 may be implemented in various types such as a computing device within a workstation and a mobile device.

The user terminal 30 may display medical image-related data stored in the medical image storage device 20 in conjunction with the medical image storage device 20. Additionally, the user terminal 30 may display a bone disease result diagnosed by the bone disease diagnosis system 10. The function may be provided through a viewer that is a dedicated program installed in the user terminal 30.

The medical image storage device 20 may store and manage captured medical images. The medical image storage device 20 may store and manage analysis results of the medical images. The medical image storage device 20 may include a database (e.g., a picture archiving and communications system(PACS) database) of a picture archiving and communications system(PACS) (hereinafter referred to as 'PACS'). The PACS may be a medical image storage and transmission system. The medical image storage device 20 may store data according to a designated data format.

The medical image storage device 20 may obtain bone disease diagnosis information (e.g., osteoporosis/osteopenia/normal condition) from the bone disease diagnosis system 10. The medical images stored in the medical image storage device 20 may include an X-ray image, a magnetic resonance imaging (MRI) image, an ultrasound image, a computed tomography (CT) image, a digital mammography (MMG) image, a digital breast tomosynthesis (DBT) image, and the like.

In the embodiment of the present disclosure, a chest X-ray image (hereinafter also simply referred to as an "X-ray image") is described as an example of the medical image, but the present disclosure is not limited thereto and may be applied according to a type of the medical image.

The bone disease diagnosis system 10 may analyze a chest X-ray (hereinafter also referred to as "CXR") image using a first artificial intelligence model (e.g., a non-osteoporosis classification model) and a second artificial intelligence model (e.g., an osteopenia classification model), and may diagnose whether there is a bone disease (e.g., whether there is osteopenia) based on a CXR analysis result.

The bone disease diagnosis system 10 according to an embodiment of the present disclosure may classify an input CXR image into osteoporosis (e.g., an osteoporosis image) or non-osteoporosis (e.g., a non-osteoporosis image) using the first artificial intelligence model, and may classify a non-osteoporosis image classified by the first artificial intelligence model into osteopenia (e.g., an osteopenia image) or normal condition (e.g., a normal condition image) using the second artificial intelligence model.

According to an embodiment of the present disclosure, the first artificial intelligence model may include a plurality of classification models specialized for each of an entire CXR image and a plurality of segmentation CXR images. For example, according to an embodiment of the present disclosure, an input entire CXR image may be segmented into the plurality of segmentation CXR images by reflecting an anatomical feature, and then the entire image and each segmented image may be classified as the osteoporosis or the non-osteoporosis based on a corresponding classification model of the first artificial intelligence model.

An artificial intelligence model according to an embodiment of the present disclosure may be generated to perform medical inference from an input medical image, and a model structure, a training data configuration, a training method, a medical inference target, and the like may be variously designed.

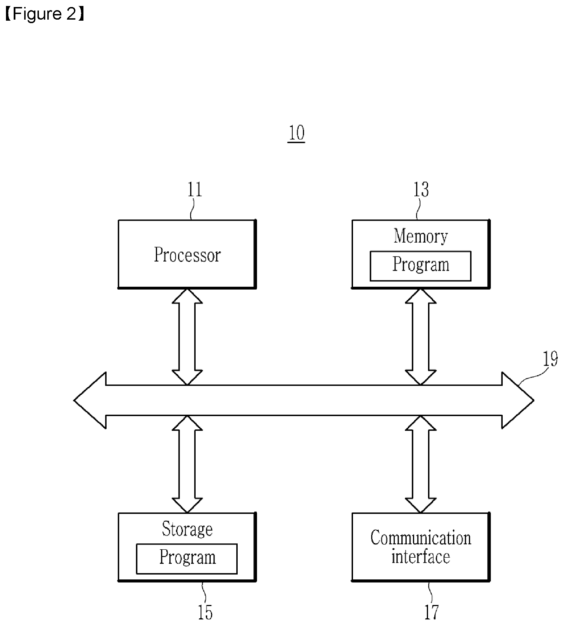

The bone disease diagnosis system 10 or a detailed device within the bone disease diagnosis system according to an embodiment of the present disclosure may be implemented as a computing device shown in FIG. 2.

Referring to FIG. 2, the bone disease diagnosis system or the detailed device (e.g., a computing device 10) within the bone disease diagnosis system may include one or more processors 11, a memory 13 loading a program performed by the processor 11, a storage 15 for storing a program and various data, a communication interface 17, and a bus 19 connecting the processor 11, the memory 13, the storage 15, and the communication interface 17. In addition, the bone disease diagnosis system 10 may further include various components.

The program may include instructions that allow the processor 11 to perform methods/operations according to various embodiments of the present disclosure when it is loaded in the memory 13. For example, the processor 11 may perform the methods/operations according to various embodiments of the present disclosure by executing the instructions. The program may include a series of computer-readable instructions grouped based on a function, and may be executed by the processor.

The processor 11 may control an overall operation of each component of the computing device 10. The processor 11 may include a single physical entity, but may also include a plurality of physical entities. The processor 11 including the plurality of physical entities may segment and process a single execution element, or may segment and process a plurality of execution elements.

The processor 11 may be configured to include at least one of a central processing unit (CPU), a micro processor unit (MPU), a micro controller unit (MCU), a graphics processing unit (GPU), and any type of processor well known in a technical field of the present disclosure. Additionally, the processor 11 may perform a calculation for at least one application or program for executing the methods/operations according to various embodiments of the present disclosure.

The memory 13 may store various data, commands, and/or information. The memory 13 may load one or more programs from the storage 15 to execute the methods/operations according to various embodiments of the present disclosure. The memory 13 may be implemented as a volatile memory such as a RAM, but a technical scope of the present disclosure is not limited thereto.

The storage 15 may store non-temporarily the program. The storage 15 may be configured to include a nonvolatile memory such as a read only memory (ROM), an erasable programmable ROM (EPROM), an electrically erasable programmable ROM (EEPROM), or a flash memory, a hard disk, a removable disk, or any type of computer-readable recording medium well known in a technical field to which the present disclosure belongs.

According to an embodiment of the present disclosure, the storage may store the first artificial intelligence model including the plurality of classification models, the second artificial intelligence model, and a trained parameter (e.g., a weight or a bias) of each artificial intelligence model. In this case, the first and second artificial intelligence models may be a training model having a neural network structure that trains a relationship between an input image and a label.

The communication interface 17 may support wired and wireless Internet communication of the computing device 10. In addition, the communication interface 17 may support various communication methods other than Internet communication. To this end, the communication interface 17 may be configured to include a communication module well known in the art of the present disclosure.

The bus 19 may provide a communication function between components of the computing device 10. The bus 19 may be implemented as various types of buses such as an address bus, a data bus, and a control bus.

A computer program may include instructions executed by the processor 11, may be stored in a non-transitory computer-readable storage medium, and the instructions may allow the processor 11 to execute an operation according to an embodiment of the present disclosure. The computer program may be downloaded over a network or may be sold in a product form.

The computer program according to an embodiment of the present disclosure may include instructions for collecting a dataset for pre-training the artificial intelligence model and training the artificial intelligence model using the collected dataset. The computer program may include instructions for preprocessing input data and training the artificial intelligence model using the preprocessed input data.

A functional block indicated as "module" of the detailed device of the bone disease diagnosis system 10 described below may be implemented by cooperation between the processor 11, which is a detailed configuration of the computing device shown in FIG. 2, and the program included in the memory 13 (or the storage 15) that is the detailed configuration of the computing device shown in FIG. 2.

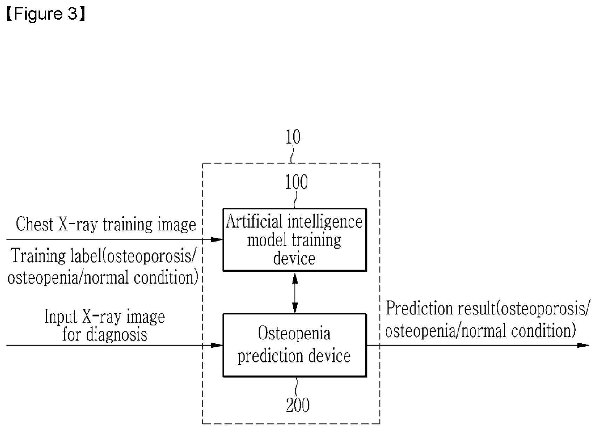

Hereinafter, the bone disease diagnosis system 10 according to a first embodiment of the present disclosure will be described with reference to FIGS. 3 to 10.

FIG. 3 is a view showing the bone disease diagnosis system 10 according to an embodiment of the present disclosure.

Referring to FIG. 3, the bone disease diagnosis system 10 according to the embodiment of the present disclosure may include an artificial intelligence model training device 100 and an osteopenia prediction device 200.

The artificial intelligence model training device 100 may include the first artificial intelligence model that classifies the input CXR image as the osteoporosis or the non-osteoporosis, and the second artificial intelligence model that classifies the non-osteoporosis image as the osteopenia or the normal condition.

The osteopenia prediction device 200 may initially classify an input CXR image for diagnosis as the osteoporosis or the non-osteoporosis based on the first artificial intelligence model trained by the artificial intelligence model training device 100, and may classify the initially classified non-osteoporosis image as the normal condition or the osteopenia based on the second artificial intelligence model trained by the artificial intelligence model training device 100.

For example, the osteopenia prediction device 200 may output a prediction result as "osteoporosis" if the input CXR image for diagnosis is classified as the osteoporosis by the first artificial intelligence model of the artificial intelligence model training device 100, and if the input CXR image for diagnosis is classified as the non-osteoporosis by the first artificial intelligence model, the osteopenia prediction device 200 may output the prediction result as "normal condition" or "osteopenia" based on the second artificial intelligence model trained by the artificial intelligence model training device 100.

According to an embodiment, the first artificial intelligence model and the second artificial intelligence model may be included in the osteopenia prediction device 200.

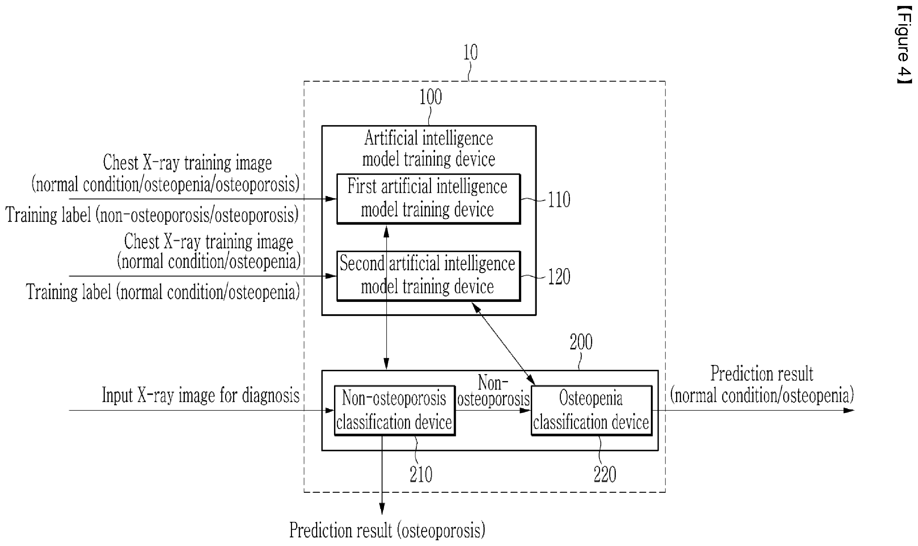

FIG. 4 is a view showing a detailed configuration of each of the artificial intelligence model training device 100 and the osteopenia prediction device 200 according to a first embodiment of the present disclosure.

Referring to FIG. 4, the artificial intelligence model training device 100 according to the first embodiment of the present disclosure may include a first artificial intelligence model training device 110 and a second artificial intelligence model training device 120.

The first artificial intelligence model training device 110 may train the first artificial intelligence model including the plurality of classification models. Each classification model may be an artificial intelligence model corresponding to the entire CXR image (hereinafter also briefly referred to as an "entire image") or the plurality of segmentation CXR images (hereinafter also briefly referred to as "segmented images") obtained by segmenting the entire CXR image into anatomical regions (e.g., an entire chest, left clavicle-scapula, right clavicle-scapula, a cervical vertebra, and thoracic vertebra/lumbar vertebra).

According to the first embodiment of the present disclosure, the first artificial intelligence model training device 110 may receive the entire CXR image (i.e., the entire image) of each of a normal person, a patient with the osteopenia, and a patient with the osteoporosis as a training image.

For example, the first artificial intelligence model training device 110 may receive the entire image of the normal person, the patient with the osteopenia, or the patient with the osteoporosis and a training label (osteoporosis/non-osteoporosis) corresponding thereto as the training data. Here, the training label for the "non-osteoporosis" may be a classification that is not the osteoporosis and may be a training label corresponding to images of the normal person and the patient with the osteopenia, and the training label for the "osteoporosis" may be a training label corresponding to the image of the patient with the osteoporosis.

As described below, the first artificial intelligence model training device 110 may segment the received entire image into a plurality of segmented images corresponding to the anatomical region, may generate a training dataset based on the entire image or the segmented image and the received training label (osteoporosis/non-osteoporosis), and may train a corresponding classification model using each training dataset.

The second artificial intelligence model training device 120 may generate a training dataset based on the entire CXR image (i.e., the entire image) of the normal person or the patient with the osteopenia and a training label (normal condition/osteopenia) corresponding thereto, and then may train the second artificial intelligence model using each training dataset.

Thus, according to the first embodiment of the present disclosure, the first artificial intelligence model training device 110 for training the osteoporosis or the non-osteoporosis may use the entire image and the segmented image as the training data, but the second artificial intelligence model training device 120 for training the osteopenia or the normal condition may use only the entire image as the training data.

Because a difference between the normal condition and the osteopenia is smaller than a difference between the non-osteoporosis and the osteoporosis, there may be a limitation in precisely distinguishing even a minute difference between the normal condition and the osteopenia with only information from a specific region based on the segmented image. Accordingly, the second artificial intelligence model training device 120 according to the first embodiment of the present disclosure may use the entire image as the training data to consider subtle contrast of a bone in the entire image, a bone quality pattern of an entire body, and overall conditioning of an image according to a photographing condition.

The osteopenia prediction device 200 according to the first embodiment of the present disclosure may include a non-osteoporosis classification device 210 and an osteopenia classification device 220.

The non-osteoporosis classification device 210 may classify the input CXR image for diagnosis into the osteoporosis or the non-osteoporosis based on the first artificial intelligence model trained by the first artificial intelligence model training device 110. The non-osteoporosis classification device 210 may output a prediction result of the "osteoporosis" if the input CXR image for diagnosis is classified as the osteoporosis by the first artificial intelligence model of the artificial intelligence model training device 100, and the non-osteoporosis classification device 210 may transfer the image classified as the non-osteoporosis to the osteopenia classification device 220 if the input CXR image for diagnosis is classified as the non-osteoporosis by the first artificial intelligence model.

The osteopenia classification device 220 may classify the non-osteoporosis image classified by the non-osteoporosis classification device 210 as the osteopenia or the normal condition based on the second artificial intelligence model trained by the second artificial intelligence model training device 120, and may output the classification result as a prediction result.

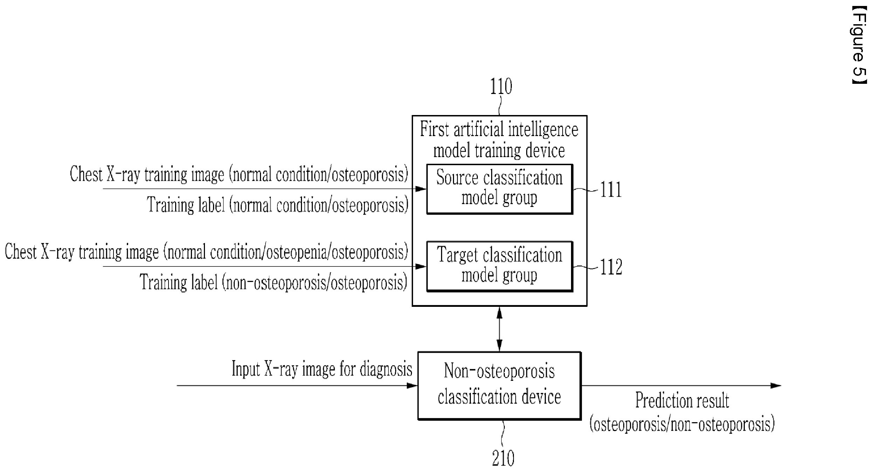

Next, the first artificial intelligence model training device 110 and the non-osteoporosis classification device 210 according to the first embodiment of the present disclosure will be described in detail with reference to FIG. 5.

The first artificial intelligence model training device 110 may include a source classification model group 111 including a plurality of source classification models (e.g., artificial intelligence models) and a target classification model group 112 including a plurality of target classification models.

Each source classification model and each target classification model may be an artificial intelligence model corresponding to the entire CXR image or the plurality of segmentation CXR images segmented corresponding to each anatomical region.

The first artificial intelligence model training device 110 may receive the entire CXR image (i.e., the entire image) of the normal person, the patient with the osteopenia, or the patient with the osteoporosis as the training image.

As described below, the first artificial intelligence model training device 110 may train the source classification model group 111 based on a training image and a training label (normal condition/osteoporosis) of each of the normal person and the patient with the osteoporosis that are relatively easy to train, may perform transfer learning for a hidden layer parameter (e.g., a weight or a bias), which is a training result of the source classification model group 111, to the target classification model group 112, and then may train the target classification model group 112 based on a training image and a training label (non-osteoporosis/osteoporosis) of each of the normal person, the patient with the osteopenia, and the patient with the osteoporosis that are relatively difficult to train.

For example, the first artificial intelligence model training device 110 according to the first embodiment of the present disclosure may receive the entire CXR image (the entire image) of the normal person or the patient with the osteoporosis and a training label (normal condition/osteoporosis) corresponding thereto as source training data for training the source classification model group 111. In addition, the first artificial intelligence model training device 110 may receive the entire CXR image (the entire image) of the normal person, the patient with the osteopenia, or the patient with the osteoporosis and a training label (non-osteoporosis/osteoporosis) corresponding thereto as target training data for training the target classification model group 112. For example, the first artificial intelligence model training device 110 according to the first embodiment of the present disclosure may receive a training label (non-osteoporosis/osteoporosis) segmented into two categories for the entire CXR image of the normal person, the patient with the osteopenia, or the patient with the osteoporosis as a target training label for training the target classification model group 112.

Thus, according to the first embodiment of the present disclosure, because the first artificial intelligence model training device 110 trains the target classification model group 112 through a target training label (non-osteoporosis/osteoporosis) segmented into two categories, there may be an advantage in which even a relatively small number of images of each of the normal person, the patient with the osteopenia, and the patient with the osteoporosis sufficiently trains the target classification model group 112.

The source classification model and the target classification model used in the first embodiment of the present disclosure may include a deep neural network structure. A deep neural network (DNN), a convolutional neural network (CNN), a recurrent neural network (RNN), a restructured Boltzmann machine (RBM), a deep belief network (DBM), a Q network, a long short-term memory (LSTM), a gated recurrent unit (GRU), a transformer, a capsule network, or the like may be used as a deep neural network algorithm, and the present disclosure is not limited thereto and another artificial intelligence model may be used.

In this case, according to the first embodiment of the present disclosure, a plurality of source classification models and a plurality of target classification models respectively included in the source classification model group and the target classification model group may use an artificial intelligence model having the same neural network structure. However, the present disclosure is not limited thereto, and classification models having different neural network structures for the source classification model group and the target classification model group may be used.

Referring to FIG. 5, the non-osteoporosis classification device 210 may receive an input CXR image for diagnosis (hereinafter also referred to as a "target image"), and may classify the received target image as the osteoporosis or the non-osteoporosis based on the target classification model group 112 trained by the first artificial intelligence model training device 110. For example, the non-osteoporosis classification device 210 may segment the received target image into the plurality of segmented images corresponding to the anatomical region, and may classify each target classification model into the osteoporosis or the non-osteoporosis based on the target classification model in which the training is completed corresponding to each of the entire image and the segmented image. Then, the non-osteoporosis classification device 210 may finally classify a plurality of inferred bone disease classification results into the osteoporosis or the non-osteoporosis using an ensemble algorithm.

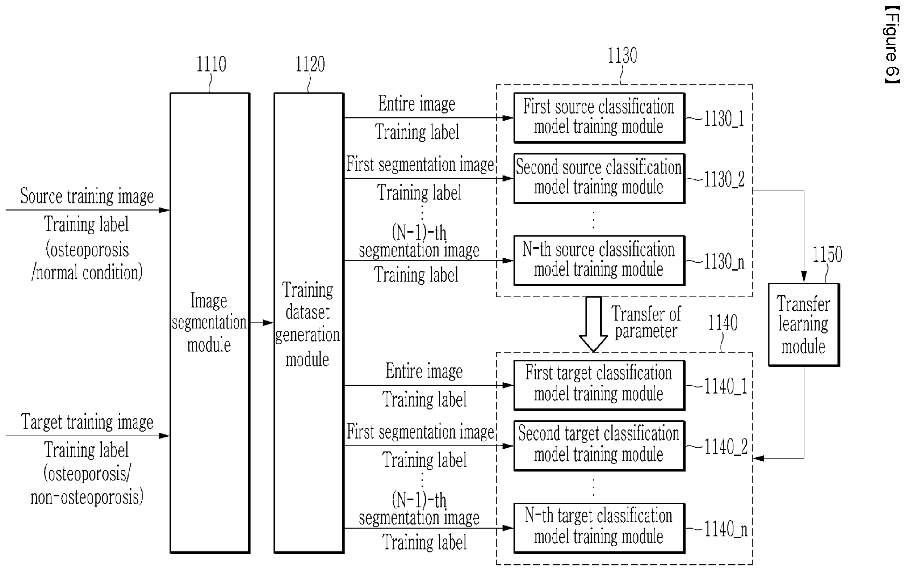

FIG. 6 is a view showing a detailed configuration of the first artificial intelligence model training device 110 according to the first embodiment of the present disclosure.

Referring to FIG. 6, the first artificial intelligence model training device 110 according to the first embodiment of the present disclosure may include an image segmentation module 1110, a training dataset generation module 1120, a plurality of source classification model training modules 1130, a plurality of target classification model training modules 1140, and a transfer learning module 1150.

The image segmentation module 1110 may receive a source training image that is the entire CXR image and a source training label (normal condition/osteoporosis) of each of the normal person and the patient with the osteoporosis, and may segment the source training image into images respectively corresponding to anatomical regions. Additionally, the image segmentation module 1110 may receive a target training image that is the entire CXR image of each of the normal person, the patient with the osteopenia, and the patient with the osteoporosis and a target training label (non-osteoporosis/osteoporosis) corresponding thereto, and may segment the target training image into the images respectively corresponding to the anatomical regions.

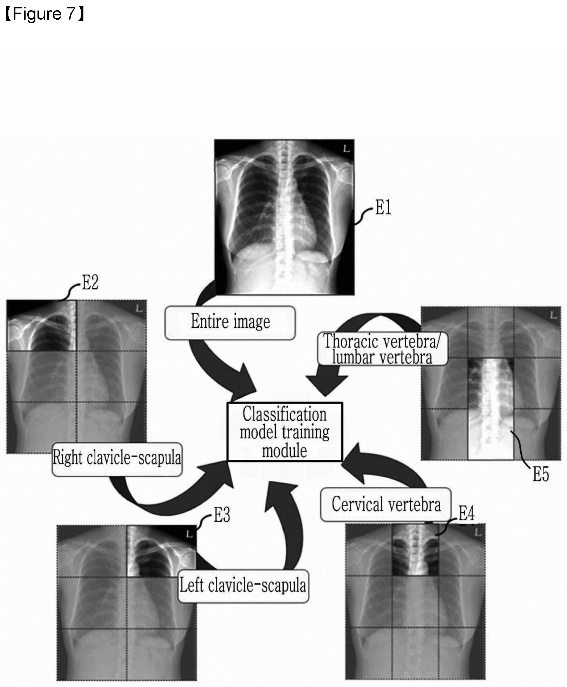

FIG. 7 is a view showing an example of each of the CXR image segmented by the image segmentation module 1110 and the entire CXR image according to the first embodiment of the present disclosure.

Referring to FIG. 7, the image segmentation module 1110 according to the first embodiment of the present disclosure may generate the segmented CXR image by cropping the entire CXR image by the anatomical region. For example, the image segmentation module 1110 may crop the entire CXR image by the anatomical region to generate the segmented image corresponding to each of an entire chest image E1, a right clavicle-scapula image E2, a left clavicle-scapula image E3, a cervical vertebra image E4, and a thoracic vertebra/lumbar vertebra image E5. Thus, according to the first embodiment of the present disclosure, because a separate artificial intelligence model that detects a region of the bone or performs segmentation in the entire CXR image is not used and an original CXR image is equally segmented and then a piece image including each anatomical region is used as each training image, there may be an advantage in which a preprocessing time of data used in the classification model and a computing resource are saved. Conventionally, a high-resolution original image was first reduced to 1k (1024) pixels to be input, but according to the first embodiment of the present disclosure, the segmented image may be generated for each anatomical region in a state in which a resolution of an original image is significantly reduced to prevent damage to the resolution of the original image. As a result, the first embodiment of the present disclosure may have an effect of improving accuracy of the training and the classification. In addition, the first embodiment may intensively train the region of the bone by cropping the entire image by the anatomical region.

However, the present disclosure is not limited to a method of equally segmenting and cropping the entire image into the images respectively corresponding to the anatomical regions, and various image segmentation techniques such as use of a segmentation model may be used in the present disclosure.

In the first embodiment of the present disclosure, each of the entire chest image E1, the right clavicle-scapula E2, the left clavicle-scapula E3, the cervical vertebra E4, and the thoracic vertebra/lumbar vertebra E5 that is the segmented image may be used, but the present disclosure is not limited thereto, and the entire CXR image and images corresponding to some anatomical regions (e.g., a right clavicle-scapula image and a left clavicle-scapula image) may be used, or only the segmented images may be used instead of the entire image.

The training dataset generation module 1120 may generate a plurality of source training datasets based on the entire CXR image and the source training label (normal condition/osteoporosis) related thereto that are input as the source training image and the plurality of segmented images segmented by the image segmentation module 1110. Additionally, the training dataset generation module 1120 may generate a plurality of target training datasets based on the entire CXR image and the target training label (non-osteoporosis/osteoporosis) related thereto that are input as the target training label (non-osteoporosis/osteoporosis) and the plurality of segmented images segmented by the image segmentation module 1110.

The source classification model training module 1130 may train the source classification model group 111 based on a source training image and a source training label (normal condition/osteoporosis) of each of the normal person and the patient with the osteoporosis that are relatively easy to train. The source classification model training module 1130 may include a plurality of source classification model training modules 1130-1, 1130-2, ..., 1130_n that respectively train the plurality of source training datasets. For example, a first source classification model training module 1130_1 may train a first source classification model using an entire source training image and a source training label that are generated by the training dataset generation module 1120. A second source classification model training module 1130_2 may train a second source classification model (e.g., an artificial intelligence model) using a first segmented image and a source training label that are generated by the training dataset generation module 1120.

The transfer learning module 1150 may perform transfer learning for the hidden layer parameter that is the training result of the source classification model group 111 to the target classification model group 112.

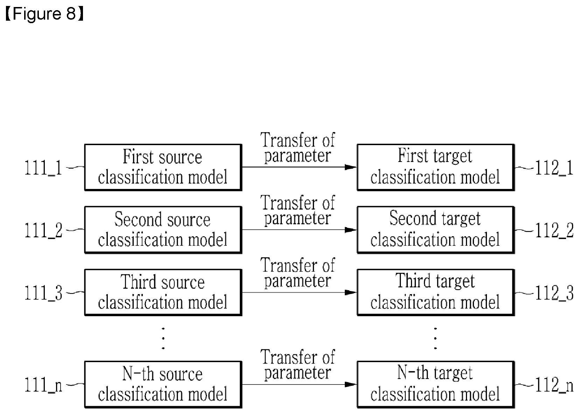

FIG. 8 is a view illustrating that the transfer learning module 1150 performs transfer learning for the hidden layer parameter that is the training result of the source classification model group 111 to the target classification model group 112.

Referring to FIG. 8, the transfer learning module 1150 sets at least some of the parameters of the source classification model by transferring them to the corresponding parameters of the target classification model. For example, the transfer learning module 1150 may transfer and set at least some of parameters of the first source classification model to a parameter of a first target classification model, and may transfer and set at least some of parameters of the second source classification model to a parameter of a second target classification model. In the same way, the transfer learning module 1150 may transfer and set at least some of parameters of an n-th source classification model to a parameter of an n-th target classification model.

Referring back to FIG. 6, the target classification model training module 1140 may train the target classification model group 112 to which the hidden layer parameter, which is the training result of the source classification model group 111, is transferred based on a target training image and a target training label (non-osteoporosis/osteoporosis) of each of the normal person, the patient with the osteopenia, and the patient with the osteoporosis.

The target classification model training module 1140 may include a plurality of target classification model training modules 1140-1, 1140-2, ..., 1140_n that respectively train the plurality of target training datasets. For example, a first target classification model training module 1140_1 may train a first target classification model using an entire target training image and a target training label that are generated by the training dataset generation module 1120. A second target classification model training module 1140_2 may train a second target classification model using a first segmented image and a target training label that are generated by the training dataset generation module 1120.

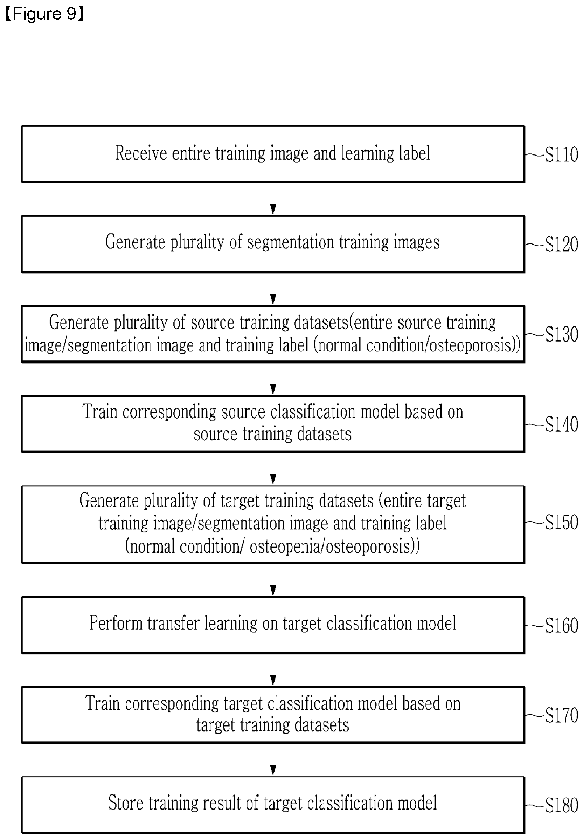

Next, a classification model training method of the first artificial intelligence model training device 110 according to the first embodiment of the present disclosure will be described in detail with reference to FIG. 9.

The first artificial intelligence model training device 110 may receive source training image/source training label and target training image/target training label as the training data (S110). For example, the first artificial intelligence model training device 110 may receive the source training image, which is the entire CXR image of each of the normal person and the patient with the osteoporosis, and the source training label (normal condition/osteoporosis) corresponding thereto, and may receive the target training image that is the entire CXR image of each of the normal person, the patient with the osteopenia, and the patient with the osteoporosis and the target training label (non-osteoporosis/osteoporosis) corresponding thereto.

Thereafter, the image segmentation module 1110 of the first artificial intelligence model training device 110 may segment the received source training image and target training image into the plurality of segmented images corresponding to the anatomical region, respectively (S120). According to the first embodiment of the present disclosure, the image segmentation module 1110 may generate the segmented CXR image by cropping the input entire CXR image by the anatomical region.

The training dataset generation module 1120 may generate the plurality of source training datasets based on the entire CXR image and the source training label (normal condition/osteoporosis) corresponding thereto that are input as the source training image and the plurality of segmented images segmented by the image segmentation module 1110 (S130).

The source classification model training module 1130 may train a corresponding source classification model using each of the plurality of source training datasets including the source training image and the source training label (normal condition/osteoporosis) of each of the normal person and the patient with the osteoporosis that are relatively easy to train as training data for the corresponding source classification model (S140).

The training dataset generation module 1120 may generate the plurality of target training datasets based on the entire CXR image and the target training label (non-osteoporosis/osteoporosis) corresponding thereto that are input as the target training label (non-osteoporosis/osteoporosis) and the plurality of segmented images segmented by the image segmentation module 1110 (S150).

The transfer learning module 1150 may perform transfer learning on the target classification model based on the source classification model in which the training is completed (S160). For example, the transfer learning module 1150 may perform transfer learning on a corresponding target classification model based on an artificial intelligence neural network parameter of the source classification model in which the training is completed.

The target classification model training module 1140 may train a corresponding target classification model for the transferred target classification model using each of the plurality of target training datasets including the target training image and the target training label (non-osteoporosis/osteoporosis) of each of the normal person, the patient with the osteopenia, and the patient with the osteoporosis as training data for the corresponding target classification model (S170).

The target classification model training module 1140 may store a training parameter (e.g., a weight or a bias) that is a training result of the target classification model based on the target training dataset in a memory or a storage (S180).

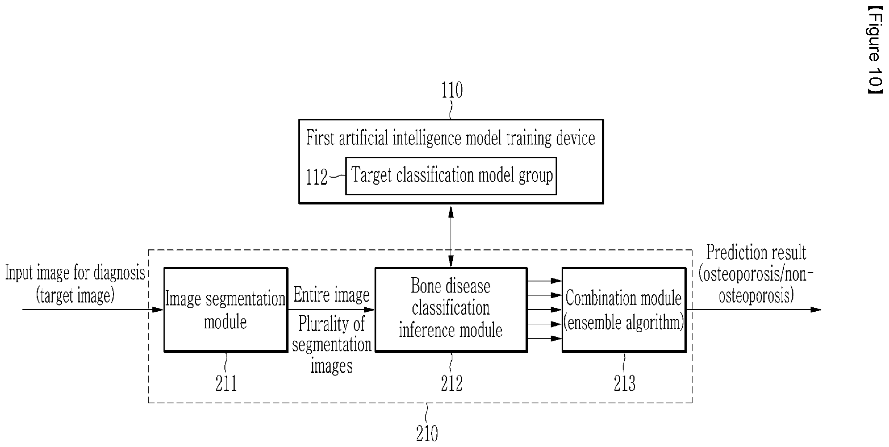

Next, the non-osteoporosis classification device 210 according to the first embodiment of the present disclosure will be described with reference to FIG. 10.

FIG. 10 is a view showing a detailed configuration of the non-osteoporosis classification device 210 according to the first embodiment of the present disclosure.

Referring to FIG. 10, the non-osteoporosis classification device 210 according to the first embodiment of the present disclosure may include an image segmentation module 211, a bone disease classification inference module 212, and a combination module 213.

The image segmentation module 211 may receive a input CXR image for diagnosis (hereinafter also referred to as the "target image"), and may segment an entire target image into images respectively corresponding to anatomical regions. In this case, the image segmentation module 211 of the non-osteoporosis classification device 210 may segment the entire image into the images respectively corresponding to the anatomical regions using the same segmentation method as that of the image segmentation module 1110 of the first artificial intelligence model training device 110.

The bone disease classification inference module 212 may infer a bone disease classification result for each image (e.g., the entire target image or a plurality of segmented images segmented by the image segmentation module 211) based on a corresponding target classification model among a plurality of target classification model groups 112 trained by the first artificial intelligence model training device 110. In this case, the classification result inferred by the bone disease classification inference module 212 may be a logit value or a probability value.

The combination module 213 may combine a plurality of bone disease classification results inferred by the bone disease classification inference module 212 using an ensemble algorithm to diagnose whether there is a final bone disease (osteoporosis/non-osteoporosis).

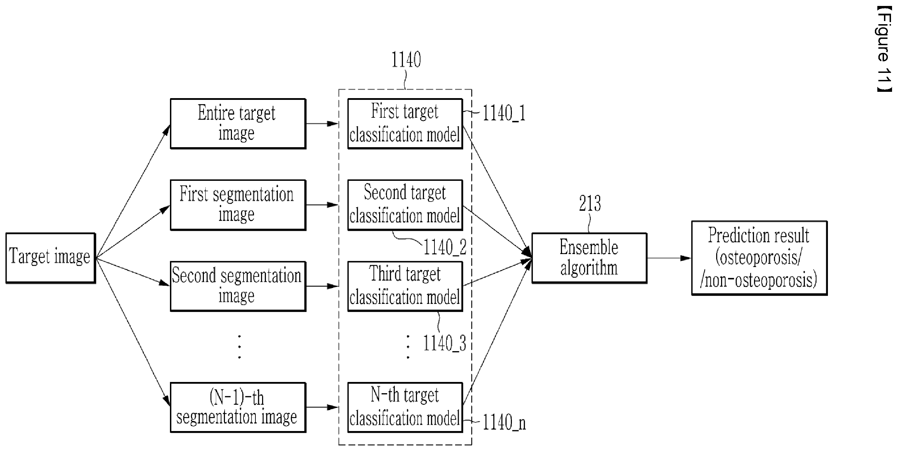

FIG. 11 is a view showing a combination process performed in the combination module 213 according to the first embodiment of the present disclosure.

Referring to FIG. 11, the combination module 213 may combine (or synthesize) the plurality of bone disease classification results using the ensemble algorithm. For example, the combination module 213 may average logit values or probability values that are bone disease classification result values inferred from each classification model, and may compare the average value with a reference value to determine whether there is a bone disease (osteoporosis/non-osteoporosis). In this case, according to the first embodiment of the present disclosure, the combination module 213 may determine whether there is the bone disease (osteoporosis/non-osteoporosis) by calculating an average value for only the remaining results excluding one or more of the results when the plurality of bone disease classification results are combined. According to the first embodiment of the present disclosure, various techniques other than the average may be applied as an ensemble technique. For example, any one of voting, bagging, boosting, and stacking, or a combination thereof, may be used as an ensemble technique.

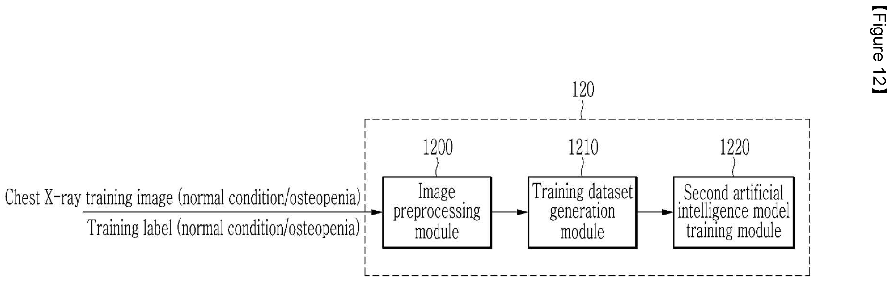

Next, the second artificial intelligence model training device 120 and a second artificial intelligence model training method according to the first embodiment of the present disclosure will be described in detail with reference to FIGS. 12 to 14.

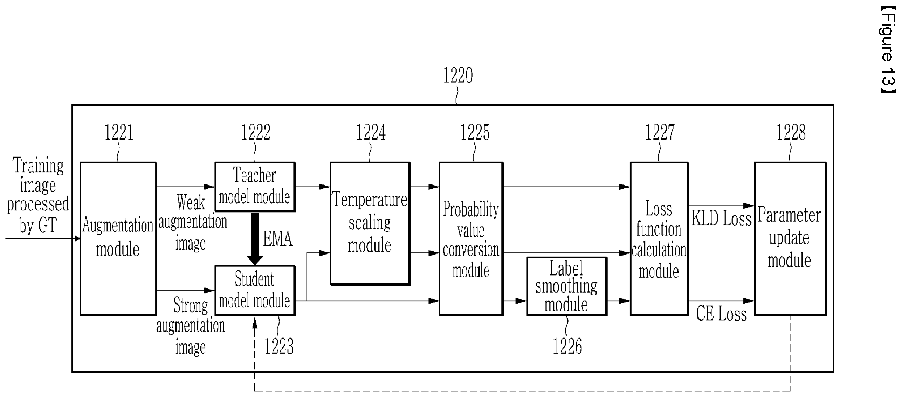

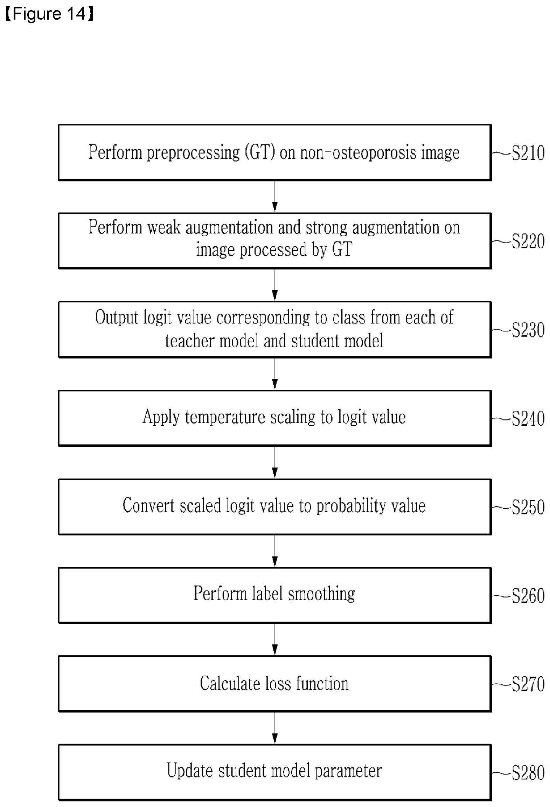

FIG. 12 is a view showing the second artificial intelligence model training device 120 according to the first embodiment of the present disclosure, FIG. 13 is a view showing a detailed configuration of a second artificial intelligence model training module 1220 according to the first embodiment of the present disclosure, and FIG. 14 is a view showing the second artificial intelligence model training method according to the first embodiment of the present disclosure.

Referring to FIG. 12, the second artificial intelligence model training device 120 according to the first embodiment of the present disclosure may include an image preprocessing module 1200, a training dataset generation module 1210, and the second artificial intelligence model training module 1220.

The image preprocessing module 1200 may perform preprocessing on a non-osteoporosis image (normal condition or an osteopenia image) that is a training CXR image (S210).

Because a CXR image equipment includes a different image storage algorithm depending on a manufacturer or an equipment version, a dataset may be constructed only with an image photographed with an equipment of a specific manufacturer so that training of the model is overfitted only to the image of the manufacturer. Accordingly, in the first embodiment of the present disclosure, the preprocessing may be performed on the training CXR image using grayscale truncation (GT) that is a technique of cutting a part of a pixel value from a grayscale image.

According to the first embodiment of the present disclosure, a detailed method of the preprocessing of the grayscale truncation (GT) performed in the image preprocessing module 1200 is as follows.

### (1) Performing primary normalization

After the CXR image is segmented into regions (e.g., pixels) with a matrix form having a predetermined height and a predetermined width, each pixel value may be normalized between 0 and 1 using Mathematical expression 1 below. Through the primary normalization, a convergence speed of an optimization algorithm such as a gradient descent method may be improved. $\frac{x - Min \left(X\right)}{Max \left(X\right) - Min \left(X\right)}$

In Mathematical expression 1 above, Max(X) may represent a maximum pixel value, and Min(X) may represent a minimum pixel value.

### (2) Perform secondary normalization

After a central region is obtained from the image to which a primary normalization algorithm is applied using Mathematical expression 2 below, the secondary normalization may be performed on an entire pixel value (X'_{i,j}) based on a minimum value (Min(Area_{c})) and a maximum value (MAX(Area_{c})) obtained from the central region using Mathematical expression 3 below. ${Area}_{c} = \left\{{pixel}_{i , j}\left|i∈\left[\frac{height}{4}, \frac{3 * height}{4}\right],j∈\left[\frac{width}{4}, \frac{3 * width}{4}\right]\right.\right\}$ ${x}_{i , j}^{′} = \left\{\begin{matrix}Min \left({Area}_{c}\right) , if {x}_{i , j}^{′} = \left〈Min\left({Area}_{c}\right)\right. \\ {x}_{i , j}^{′} , if Min \left({Area}_{c}\right) \left〈{x}_{i , j}^{′}\left〈Max\left({Area}_{c}\right)\right.\right. \\ \left.Max\left({Area}_{c}\right),if {x}_{i , j}^{′}\right〉 = Max \left({Area}_{c}\right)\end{matrix}\right.$

Referring to Mathematical expression 3 above, if the pixel value (X'_{i,j}) is less than or equal to the minimum value (Min(Area_{c})), the pixel value may be considered as a very dark value and may be adjusted to the minimum value (Min(Area_{c})), and if the pixel value (X'_{i,j}) is greater than or equal to the maximum value (Max(Area_{c})), the pixel value may be considered to be excessively bright and may be adjusted to the maximum value (Max(Area_{c})).

### (3) Performing tertiary normalization

MinMax scaling may be performed on the secondary normalization image by referring to Mathematical expression 4 below. $\frac{x - Min \left(X\right)}{Max \left(X\right) - Min \left(X\right)}$

The training dataset generation module 1210 may generate the training dataset based on a training image (e.g., an image of the normal person or the patient with the osteopenia) processed by the grayscale truncation (GT) that is performed by the image preprocessing module 1200 and a training label (normal condition/osteopenia) related thereto.

The second artificial intelligence model training module 1220 may train the second artificial intelligence model using the training image processed by the grayscale truncation (GT) performed in the image preprocessing module 1200.

According to the first embodiment of the present disclosure, a knowledge distillation model may be used as the second artificial intelligence model. The knowledge distillation model may refer to a deep learning technique that improves performance of a small model by transferring knowledge from a large model (e.g., a teacher model) to a small model (e.g., a student model).

Referring to FIG. 13, the second artificial intelligence model training module 1220 according to the first embodiment of the present disclosure may include an augmentation module 1221, a teacher model module 1222, a student model module 1223, a temperature scaling module 1224, a probability value conversion module 1225, a label smoothing module 1226, a loss function calculation module 1227, and a parameter update module 1228.

The augmentation module 1221 may perform weak augmentation and strong augmentation on the training CXR image processed by the GT (S220).

In the first embodiment of the present disclosure, the weak augmentation may mean a deformation relatively similar to that of the original, and the strong augmentation may mean a stronger deformation of the original. A technique such as Horizontal flip, Resize, or Normalize may be used as a method of the weak augmentation, and a technique such as Blur, MotionBlur, MedianBlur, ShiftScaleRotate, GridDistortion, ElasticTransform, Optical Distortion, RandomGamma, or GridDropout may be used as a method of the strong augmentation.

A weak augmentation image and a strong augmentation image performed in the augmentation module 1221 may be input to the teacher model module 1222 and the student model module 1223, respectively.

According to the first embodiment of the present disclosure, the teacher model module 1222 receiving weak augmentation datas an input may train relatively easily the weak augmentation data, whereas the student model module 1223 receiving strong augmentation datas an input may have difficulty in the training because the data is in a strongly deformed state. However, because knowledge of the teacher model module 1222 that easily trains the same data is transferred to the student model module 1223 through an exponential moving average (EMA) technique described later, the student model module 1223 may effectively train even data including complex and various deformations. Accordingly, robustness against data including a noise may be secured, generalization performance may be improved, and feature representation ability of the model may also be strengthened.

The teacher model module 1222 may receive the weak augmentation image performed by the augmentation module 1221 to output a logit value for a class, and the student model module 1223 may receive the strong augmentation image performed by the augmentation module 1221 to output a logit value for the class (S230).

Here, the "class" may refer to a category to which the data belongs, and two classes of the osteopenia or the normal condition may be used in the second artificial intelligence model according to the first embodiment of the present disclosure. The logit value may refer to a "denormalization value" for the class predicted by an artificial neural network. For example, the logit value may correspond to an intermediate value for representing a probability of belonging to a specific class.

The teacher model module 1222 and the student model module 1223 according to the first embodiment of the present disclosure may be implemented based on a CNN artificial intelligence neural network, and for example, the teacher model module 1222 and the student model module 1223 may be implemented using a ConvNeXt model.

The temperature scaling module 1224 may apply temperature scaling to the logit values respectively output from the teacher model module 1222 and the student model module 1223 as shown in Mathematical expression 5 (S240). ${P}_{i} = \frac{exp \left({z}_{i}/T\right)}{{\sum }_{j} exp \left({z}_{j}/T\right)}$

In Mathematical expression 5 above, Pᵢ may be a scaled prediction probability of Class i, zᵢ may be an output logit of the model for Class i, and T may be a temperature factor.

The logit values respectively output from the teacher model module 1222 and the student model module 1223 may be corrected through the temperature scaling. As a value of the temperature factor (T) increases, the logit value may be strongly corrected, and in the first embodiment of the present disclosure, T may be set to be greater than 111.

Thus, if the temperature scaling module 1224 applies the temperature scaling to the logit values respectively output from the teacher model module 1222 and the student model module 1223 to correct the logit value, a probability distribution may be made smoother so that over-confidence of the model is reduced.

The probability value conversion module 1225 may apply the corrected logit value output from the temperature scaling module 1224 (i.e., a value corrected by applying the temperature scaling to the logit values respectively output from the teacher model module 1222 and the student model module 1223) to a softmax function to convert the applied logit value into a class-specific probability value between 0 and 1 (S250).

For example, the probability value conversion module 1225 may output a probability value converted from the logit value corrected by applying the temperature scaling to the logit value output from the teacher model module 1222 (hereinafter also referred to as a "first probability value") and a probability value converted from the logit value corrected by applying the temperature scaling to the logit value output from the student model module 1223 (hereinafter also referred to as a "second probability value"). Additionally, the probability value conversion module 1225 may apply the logit value output from the student model module 1223 that does not pass through the temperature scaling module 1224 (i.e., the logit value to which the temperature scaling is not applied) to the softmax function to convert the applied logit value into a probability value (hereinafter referred to as a "third probability value").

The label smoothing module 1226 may perform label smoothing on a label of the class output from the student model module 1223 using Mathematical expression 6 below (S260). ${{y}_{i}}^{LS} = {y}_{i} \left(1-α\right) + α / I$

In Mathematical expression 6 above, α may be a smoothing factor with a value between 0 and 1, i may represent the class, I may be a total number of classes, yᵢ may be the label of the class output from the student model module, and yᵢ^{LS} may be a label smoothing value of the output class.

The loss function calculation module 1227 may calculate a cross entropy loss function (CE LOSS) and a Kullback-Leibler divergence loss function (KLD LOSS) based on the probability value output from the probability value conversion module 1225 (S270).

For example, the loss function calculation module 1227 may calculate the cross entropy loss function based on the third probability value output from the probability value conversion module 1225 (i.e., a probability value obtained by applying the logit value output from the student model module 1223 that does not pass through the temperature scaling module 1224 to the softmax function).

In this case, the cross entropy loss function (CE_{Loss}) may be calculated using Mathematical expression 7 below. $CE_Loss = - {\sum }_{i = 1}^{n} {y}_{i}^{LS} log \left(\hat{{y}_{i}}\right)$

In Mathematical expression 7 above, ${y}_{i}^{LS}$ may represent a label of class i, and *ŷᵢ* may represent a probability predicted by the model for class i.

Additionally, the loss function calculation module 1227 may calculate the Kullback-Leibler divergence loss function (KLD LOSS) based on the first probability value output from the probability value conversion module 1225 (e.g., a probability value converted from the logit value corrected by applying the temperature scaling to the logit value output from the teacher model module 1222) and the second probability value (e.g., a probability value converted from the logit value corrected by applying the temperature scaling to the logit value output from the student model module 1223).

The Kullback-Leibler Divergence (KLD) loss function may be an important concept used to measure a difference between probability distributions, and KL Divergence may represent a value indicating "how much information is lost from P when P is approximated by Q" when there are two probability distributions (P and Q).

A process for calculating the KLD loss function may be applied to minimize a distribution difference between the probability values obtained from the teacher model module 1222 and the student model module 1223, and may be expressed by Mathematical expression 8 below. $KLD \left({P}_{teacher}\left‖{P}_{student}\right.\right) = {\sum }_{i = 1}^{n} {P}_{teacher}^{i} log \left(\frac{{P}_{teacher}^{i}}{{P}_{student}^{i}}\right)$

In Mathematical expression 8 above, ${P}_{teacher}^{i}$ may represent a value for class i predicted in the teacher model module by applying the temperature scaling, and ${P}_{student}^{i}$ may represent a value for class i predicted in the student model module by applying the temperature scaling.

The parameter update module 1228 may update a parameter of the student model module 1223 (S280).

For example, the parameter update module 1228 may update the parameter of the student model module 1223 based on the cross entropy loss function (CE Loss) and a KLD loss function (KLD Loss) output from the loss function calculation module 1227. In some embodiments, the parameter update module 1228 may update the parameter of the student model module 1223 by summing up the cross entropy loss function (CE Loss) and the KLD loss function (KLD Loss) output from the loss function calculation module 1227 to perform backpropagation through an optimizer.

The optimizer may be a rule (or an algorithm) that determines how to update a weight of the model in order to minimize the loss function. Stochastic Gradient Descent (SGD), Adaptive Moment Estimation (ADA), AdamW, Adagrad, RMSprop, AdaMax, or the like may be used as the optimizer, and in the first embodiment of the present disclosure, Stochastic Gradient Descent (SGD) may be used as the optimizer.

Additionally, the parameter update module 1228 may calculate an Exponential Moving Average (EMA) value for a parameter (e.g., a weight or a bias) of the teacher model module 1222 and the parameter of the student model module 1223 as shown in Mathematical expression 9 below, and may update the Exponential Moving Average (EMA) value in the student model module 1223.

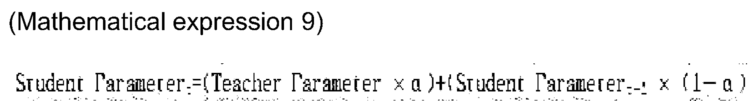

In Mathematical expression 9 above, "Teacher Parameter" may represent the parameter (e.g., the weight or the bias) of the teacher model module, "Student Parameterₜ₋₁" may represent a parameter (e.g., a weight or a bias) of the student model module, "Student Parametert" may represent a model parameter (e.g., a weight or a bias) of the transferred (or updated) student model module, and "α" may represent a smoothing factor.

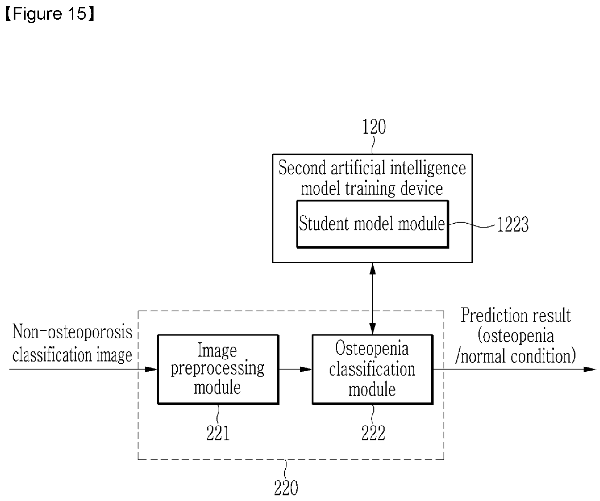

Next, the osteopenia classification device 220 according to the first embodiment of the present disclosure will be described with reference to FIG. 15.

Referring to FIG. 15, the osteopenia classification device 220 according to the first embodiment of the present disclosure may include an image preprocessing module 221 and an osteopenia classification module 222.

The image preprocessing module 221 may perform preprocessing on the non-osteoporosis image classified by the non-osteoporosis classification device 210. In this case, the image preprocessing module 221 of the osteopenia classification device 220 may perform preprocessing on the non-osteoporosis image classified by the non-osteoporosis classification device 210 using the Grayscale Truncation (GT), similar to the image preprocessing module 1200 of the second artificial intelligence model training device 120.

The osteopenia classification module 222 may finally classify the non-osteoporosis image processed by the GT in the image preprocessing module 221 into the normal condition or the osteopenia based on the student model module 1223 generated during a training process of the second artificial intelligence model training device 120, and according to an embodiment, the student model module 1223 may be included in the osteopenia classification module 222.

According to the first embodiment of the present disclosure described above, the osteopenia prediction device 200 may initially classify the input CXR image for diagnosis as the osteoporosis or the non-osteoporosis based on the first artificial intelligence model trained by the artificial intelligence model training device 100, and may classify the initially classified non-osteoporosis image as the normal condition or the osteopenia based on the second artificial intelligence model trained by the artificial intelligence model training device 100.

Thus, before the first embodiment of the present disclosure infers the osteopenia using the second artificial intelligence model specialized for classification of the osteopenia, the first embodiment of the present disclosure may first classify a state of the bone as the osteoporosis, and may infer the osteopenia or the normal condition from an image classified as the non-osteoporosis excluding the osteoporosis so that a decline in classification performance of the osteopenia due to confusion of classification between the osteoporosis and the osteopenia is prevented.

According to the first embodiment of the present disclosure, because the training of the artificial intelligence model is insufficient, there may be a case where an actual osteoporosis image is included among images classified as the non-osteoporosis through the first artificial intelligence model. In this case, a result inferred using the second artificial intelligence model may be only two such as the osteopenia and the normal condition so that accuracy of the classification is lowered.

Accordingly, after a second embodiment of the present disclosure generates the training dataset based on the entire CXR image of the patient with the osteoporosis in addition to the normal person and the patient with the osteopenia and the training label (normal condition/osteopenia/osteoporosis) corresponding thereto, the second embodiment may train the second artificial intelligence model using the training dataset and may use the result to solve the problem.

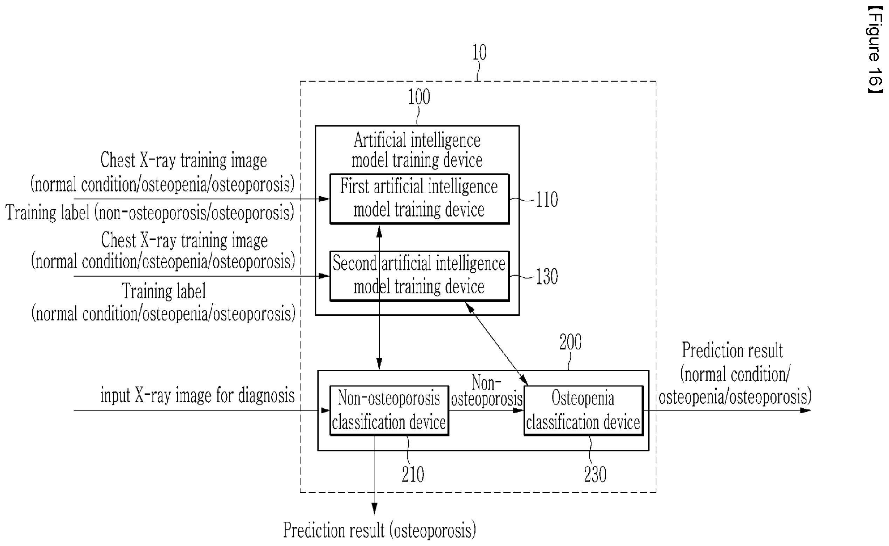

Hereinafter, an artificial intelligence model training device and an osteopenia prediction device according to the second embodiment of the present disclosure will be described with reference to FIG. 16. Apart that performs the same or similar operation as that of the component described in the first embodiment of the present disclosure among components of the artificial intelligence model training device and the osteopenia prediction device according to the second embodiment of the present disclosure uses the same reference numeral, and redundant descriptions are omitted below.

FIG. 16 is a view showing a detailed configuration of each of the artificial intelligence model training device 100 and the osteopenia prediction device 200 according to the second embodiment of the present disclosure.

Referring to FIG. 16, the artificial intelligence model training device 100 according to the second embodiment of the present disclosure may include a first artificial intelligence model training device 110 and a second artificial intelligence model training device 130.

The second artificial intelligence model training device 130 may generate a training dataset based on an entire CXR image of each of a normal person, a patient with the osteopenia, and a patient with the osteoporosis and a training label (normal condition/osteopenia/osteoporosis) corresponding thereto, and then may train an artificial intelligence model using each training dataset. For example, unlike the second artificial intelligence model training device 120 according to the first embodiment of the present disclosure, the artificial intelligence model training device 130 according to the second embodiment of the present disclosure may generate a training dataset by adding the entire CXR image of the patient with the osteoporosis and a training label (osteoporosis) corresponding thereto.

In addition, the second artificial intelligence model training device 130 according to the second embodiment of the present disclosure may include an image preprocessing module (not shown), a training dataset generation module (not shown), and a second artificial intelligence model training module (not shown), similar to the second artificial intelligence model training device 120 according to the first embodiment of the present disclosure shown in FIG. 12. However, unlike the first embodiment, the image preprocessing module of the second artificial intelligence model training device 130 according to the second embodiment of the present disclosure may perform Grayscale Truncation preprocessing on a training image that is an entire image of the normal person, the patient with the osteopenia, or the patient with the osteoporosis, and the training dataset generation module may generate a training dataset based on the training image preprocessed by the image preprocessing module and a training label of the normal person, the patient with the osteopenia, or the patient with the osteoporosis related to the training image.

A detailed configuration and a training method of the second artificial intelligence model training device 130 according to the second embodiment of the present disclosure are almost the same as those of the artificial intelligence model training device 120 according to the first embodiment of the present disclosure so that redundant descriptions are omitted below.

The osteopenia prediction device 200 according to the second embodiment of the present disclosure may include a non-osteoporosis classification device 210 and an osteopenia classification device 230.

The non-osteoporosis classification device 210 may classify the input CXR image for diagnosis into the osteoporosis or the non-osteoporosis based on the first artificial intelligence model trained by the first artificial intelligence model training device 110. The non-osteoporosis classification device 210 may output a prediction result of the "osteoporosis" if the input CXR image for diagnosis is classified as the osteoporosis by the first artificial intelligence model of the artificial intelligence model training device 100, and the non-osteoporosis classification device 210 may input the image classified as the non-osteoporosis to the osteopenia classification device 230 if the input CXR image for diagnosis is classified as the non-osteoporosis by the first artificial intelligence model.

The osteopenia classification device 230 may classify the non-osteoporosis image classified by the non-osteoporosis classification device 210 as the normal condition, the osteopenia, or the osteoporosis based on the second artificial intelligence model trained by the second artificial intelligence model training device 130, and may output the classification result as a prediction result. Accordingly, according to the second embodiment of the present disclosure, even if the osteoporosis image is incorrectly classified as the non-osteoporosis through the first artificial intelligence model, the second artificial intelligence model training device 130 may classify the incorrectly classified non-osteoporosis image as the osteoporosis.

The osteopenia prediction device 200 according to the second embodiment of the present disclosure may compare the prediction result output by the non-osteoporosis classification device 210 with the prediction result output by the osteopenia classification device 230. Thereafter, based on the comparison result, prediction for the input CXR image for diagnosis may be repeated two or more times, or a message indicating a uncertainty section or holding of the classification may be output by the processor 11. For example, if the prediction result output by the non-osteoporosis classification device 210 is "non-osteoporosis" and the prediction result output by the osteopenia classification device 230 is "osteoporosis", the osteopenia prediction device 200 may repeat prediction for the input CXR image for diagnosis two or more times.

The embodiments described above may be implemented in a form of a computer program executable through various components on a computer, and the computer program may be recorded on a computer-readable medium. In this case, the medium may include a magnetic medium such as a hard disk, a floppy disk, or a magnetic tape, an optical recording medium such as a CD-ROM or a DVD, a magneto-optical medium such as a floptical disk, and a hardware device specifically configured to store and execute a program instruction such as a ROM, a RAM, or a flash memory.

Unless there is an explicit description of an order of steps included in the method according to the embodiment of the present disclosure or a description contrary to the order of steps, the steps may be performed in a suitable order, and the present disclosure is not limited to the order of the steps. In the present disclosure, all examples or use of an exemplary term (e.g., etc.) may be simply for describing the present disclosure in detail so that a scope of the present disclosure is not limited thereto. Those skilled in the art may see that various modifications, combinations, and changes may be made within the scope The system of claims or equivalents thereof.

While this disclosure has been described in connection with what is presently considered to be practical embodiments, it should be understood that the disclosure is not limited to the disclosed embodiments, but, on the contrary, is intended to cover various modifications and equivalent arrangements included within the spirit and scope of the appended claims.

## Claims

1. A system for diagnosing osteopenia, comprising:
an artificial intelligence model training device that includes a first artificial intelligence model training device training a first artificial intelligence model that classifies an input image as osteoporosis or non-osteoporosis and a second artificial intelligence model training device training a second artificial intelligence model that classifies a non-osteoporosis image as normal condition or the osteopenia; and
an osteopenia prediction device that initially classifies an input image for diagnosis as the osteoporosis or the non-osteoporosis based on the first artificial intelligence model and classifies the initially classified non-osteoporosis image as the normal condition or the osteopenia based on the second artificial intelligence model.

2. The system of claim 1, wherein the input image is a chest X-ray image.

3. The system of claim 1, wherein the osteopenia prediction device comprises:
a non-osteoporosis classification device that classifies the input image for diagnosis as the osteoporosis or the non-osteoporosis based on the first artificial intelligence model; and
an osteopenia classification device that classifies the non-osteoporosis image classified by the non-osteoporosis classification device as the osteopenia or the normal condition based on the second artificial intelligence model.

4. The system of claim 3, wherein the first artificial intelligence model includes each of a plurality of classification models that trains an entire image or one or more segmented images obtained by segmenting the entire image into anatomical regions; and
the first artificial intelligence model training device trains a corresponding classification model among the plurality of classification models based on a training label corresponding to the entire image or the segmented image.

5. The system of claim 4, wherein the second artificial intelligence model training device trains the second artificial intelligence model based on an entire image of a normal person or a patient with the osteopenia and a training label corresponding thereto.

6. The system of claim 5, wherein the second artificial intelligence model training device comprises:
an image preprocessing module that performs grayscale truncation preprocessing on a training image that is the entire image of the normal person or the patient with the osteopenia;
a training dataset generation module that generates a training dataset based on the training image preprocessed in the image preprocessing module and a training label of the normal condition or the osteopenia related to the training image; and
a second artificial intelligence model training module that trains the second artificial intelligence model using the training dataset generated by the training dataset generation module.

7. The system of claim 6, wherein the second artificial intelligence model is a knowledge distillation model; and
the second artificial intelligence model training module comprises:
an augmentation module that performs first augmentation and second augmentation stronger than the first augmentation on the training image preprocessed in the image preprocessing module;
a teacher model module that receives the training image on which the first augmentation is performed to output a first logit value regarding a class for the osteopenia or the normal condition;
a student model module that receives the training image on which the second augmentation is performed to output a second logit value regarding the class for the osteopenia or the normal condition;
a temperature scaling module that applies temperature scaling to the first logit value and the second logit value respectively output from the teacher model module and the student model module to respectively output a first correction logit value and a second correction logit value;
a probability value conversion module that performs probability conversion on the first correction logit value and the second correction logit value respectively output from the temperature scaling module to respectively output a first probability value and a second probability value and performs probability conversion on the second logit value output from the student model module to output a third probability value;
a loss function calculation module that calculates a loss function based on the first probability value, the second probability value, and the third probability value output from the probability value conversion module; and
a parameter update module that updates a parameter of the student model module based on the loss function output from the loss function calculation module.

8. The system of claim 7, wherein the loss function calculation module calculates a cross entropy loss function based on the third probability value output from the probability value conversion module, and calculates a Kullback-Leibler divergence loss function based on the first probability value and the second probability value output from the probability value conversion module.

9. The system of claim 8, wherein the parameter update module sums the cross entropy loss function calculated in the loss function calculation module and the Kullback-Leibler divergence loss function calculated in the loss function calculation module and then performs backpropagation through an optimizer to update the parameter of the student model module.

10. The system of claim 7, wherein the parameter update module calculates an exponential moving average value for a parameter of the teacher model module and the parameter of the student model module, and updates the student model module.

11. A system for diagnosing osteopenia, comprising:
an artificial intelligence model training device that includes a first artificial intelligence model training device training a first artificial intelligence model that classifies an input image as osteoporosis or non-osteoporosis and a second artificial intelligence model training device training a second artificial intelligence model that classifies a non-osteoporosis image as normal condition, the osteopenia, or the osteoporosis; and
an osteopenia prediction device that initially classifies a input image for diagnosis as the osteoporosis or the non-osteoporosis based on the first artificial intelligence model and classifies the initially classified non-osteoporosis image as the normal condition, the osteopenia, or the osteoporosis based on the second artificial intelligence model.

12. The system of claim 11, wherein the osteopenia prediction device comprises:
a non-osteoporosis classification device that classifies the input image for diagnosis as the osteoporosis or the non-osteoporosis based on the first artificial intelligence model; and
an osteopenia classification device that classifies the non-osteoporosis image classified by the non-osteoporosis classification device as the normal condition, the osteopenia, or the osteoporosis based on the second artificial intelligence model.

13. The system of claim 11, wherein the second artificial intelligence model training device comprises:
an image preprocessing module that performs grayscale truncation preprocessing on a training image that is an entire image of a normal person, a patient with the osteopenia, or a patient with the osteoporosis;
a training dataset generation module that generates a training dataset based on the training image preprocessed in the image preprocessing module and a training label of the normal condition, the osteopenia, or the osteoporosis related to the training image; and
a second artificial intelligence model training module that trains the second artificial intelligence model using the training dataset generated by the training dataset generation module, and
the second artificial intelligence model is a knowledge distillation model.

14. The system of any one of claim 1 to claim 13, wherein the first artificial intelligence model training device comprises:
a first image segmentation module that receives a source training image that is the entire image of the normal person or the patient with the osteoporosis, segments the source training image into images respectively corresponding to anatomical regions, receives a target training image that is the entire image of the normal person, the patient with the osteopenia, or the patient with the osteoporosis, and segments the target training image into the images respectively corresponding to the anatomical regions;
a training dataset generation module that generates a plurality of source training datasets based on the source training image and a plurality of segmentation source images segmented from the source training image and generates a plurality of target training datasets based on the target training image and a plurality of segmentation target images segmented from the target training image, wherein each of the plurality of source training datasets includes a source training label labeled the normal condition or the osteoporosis with the source training image or the plurality of segmentation source images and each of the plurality of target training datasets includes a target training label labeled the non-osteoporosis or the osteoporosis with the target training image or the plurality of segmentation target images;
a plurality of source classification model training modules that perform primary training on a corresponding source classification model based on the plurality of source training datasets; and
a plurality of target classification model training modules that perform secondary training on a corresponding target classification model based on the plurality of target training datasets.

15. The system of claim 14, wherein the first artificial intelligence model training device further comprises a transfer learning module that performs transfer learning for a corresponding target classification model training module based on a hidden layer parameter that is a training result of the source classification model training module.

16. The system of claim 15, wherein the first image segmentation module crops and segments the entire image into the images respectively corresponding to the anatomical regions; and
the training image generated by the training dataset generation module includes one or more segmented images among an entire chest image, right clavicle-scapula, left clavicle-scapula, a cervical vertebra, and thoracic vertebra/lumbar vertebra.

17. The system of claim 15, wherein the non-osteoporosis classification device comprises:
a second image segmentation module that segments the input image for diagnosis into the anatomical regions to generate a plurality of segmented images;
a bone disease classification inference module that infers a bone disease classification result for each of the input image for diagnosis and the plurality of segmented images segmented by the second image segmentation module based on a corresponding classification model among the plurality of target classification models; and
a combination module that combines a plurality of bone disease classification results inferred by the bone disease classification inference module using an ensemble algorithm to diagnose whether the input image for diagnosis is an osteoporosis image or the non-osteoporosis image.

18. An artificial intelligence model training device, comprising:
a first artificial intelligence model training device that trains a first artificial intelligence model classifying an input image as osteoporosis or non-osteoporosis; and
a second artificial intelligence model training device that trains a second artificial intelligence model classifying a non-osteoporosis image as normal condition or the osteopenia,
wherein the first artificial intelligence model includes each of a plurality of classification models that trains an entire image or one or more segmented images obtained by segmenting the entire image into anatomical regions; and
the first artificial intelligence model training device trains a corresponding classification model among the plurality of classification models based on a training label corresponding to the entire image or the segmented image.

19. The artificial intelligence model training device of claim 18, wherein the second artificial intelligence model training device trains the second artificial intelligence model based on an entire image of a normal person or a patient with the osteopenia and a training label corresponding thereto.

20. The artificial intelligence model training device of claim 19, wherein the second artificial intelligence model training device comprises:
an image preprocessing module that performs grayscale truncation preprocessing on a training image that is the entire image of the normal person or the patient with the osteopenia;
a training dataset generation module that generates a training dataset based on the training image preprocessed in the image preprocessing module and a training label of the normal condition or the osteopenia related to the training image; and
a second artificial intelligence model training module that trains the second artificial intelligence model using the training dataset generated by the training dataset generation module.

21. The artificial intelligence model training device of claim 20, wherein the second artificial intelligence model is a knowledge distillation model; and
the second artificial intelligence model training module comprises:
an augmentation module that performs first augmentation and second augmentation stronger than the first augmentation on the training image preprocessed in the image preprocessing module;
a teacher model module that receives the training image on which the first augmentation is performed to output a first logit value regarding a class for the osteopenia or the normal condition;
a student model module that receives the training image on which the second augmentation is performed to output a second logit value regarding the class for the osteopenia or the normal condition;
a temperature scaling module that applies temperature scaling to the first logit value and the second logit value respectively output from the teacher model module and the student model module to respectively output a first correction logit value and a second correction logit value;
a probability value conversion module that performs probability conversion on the first correction logit value and the second correction logit value respectively output from the temperature scaling module to respectively output a first probability value and a second probability value and performs probability conversion on the second logit value output from the student model module to output a third probability value;
a loss function calculation module that calculates a loss function based on the first probability value, the second probability value, and the third probability value output from the probability value conversion module; and
a parameter update module that updates a parameter of the student model module based on the loss function output from the loss function calculation module.

22. The artificial intelligence model training device of claim 21, wherein the loss function calculation module calculates a cross entropy loss function based on the third probability value output from the probability value conversion module, and calculates a Kullback-Leibler divergence loss function based on the first probability value and the second probability value output from the probability value conversion module.

23. The artificial intelligence model training device of claim 22, wherein the parameter update module sums the cross entropy loss function calculated in the loss function calculation module and the Kullback-Leibler divergence loss function calculated in the loss function calculation module and then performs backpropagation through an optimizer to update the parameter of the student model module.

24. The artificial intelligence model training device of any one of claim 18 to claim 23, wherein the second artificial intelligence model training device trains the second artificial intelligence model to classify the non-osteoporosis as the normal condition, the osteopenia, or the osteoporosis.

25. A method for diagnosing osteopenia from an input image for diagnosis, comprising:
training a first artificial intelligence model that classifies an input image as osteoporosis or non-osteoporosis;
training a second artificial intelligence model that classifies an input non-osteoporosis image as normal condition or the osteopenia;
initially classifying the input image for diagnosis as the osteoporosis or the non-osteoporosis based on the first artificial intelligence model; and
classifying the initially classified non-osteoporosis image as the normal condition or the osteopenia based on the second artificial intelligence model.

26. The method of claim 25, wherein the first artificial intelligence model includes each of a plurality of classification models that trains an entire image or one or more segmented images obtained by segmenting the entire image into anatomical regions; and
the training of the first artificial intelligence model trains a corresponding classification model among the plurality of classification models based on a training label corresponding to the entire image or the segmented image.

27. The method of claim 26, wherein the training of the second artificial intelligence model comprises:
performing grayscale truncation preprocessing on a training image that is an entire image of a normal person or a patient with the osteopenia;
generating a training dataset based on the preprocessed training image and a training label of the normal condition or the osteopenia related to the training image; and
training the second artificial intelligence model using the training dataset generated by the training dataset generation module.

28. The method of claim 27, wherein the second artificial intelligence model is a knowledge distillation model; and
the training of the second artificial intelligence model comprises:
performing first augmentation and second augmentation stronger than the first augmentation on the preprocessed training image;
receiving, by a teacher model module, the training image on which the first augmentation is performed to output a first logit value regarding a class for the osteopenia or the normal condition;
receiving, by a student model module, the training image on which the second augmentation is performed to output a second logit value regarding the class for the osteopenia or the normal condition;
applying temperature scaling to the first logit value and the second logit value respectively output from the teacher model module and the student model module to respectively output a first correction logit value and a second correction logit value;
performing probability conversion on the first correction logit value and the second correction logit value respectively to output a first probability value and a second probability value;
performing probability conversion on the second logit value output from the student model module to output a third probability value;
calculating a loss function based on the first probability value, the second probability value, and the third probability value; and
updating a parameter of the student model module based on the calculated loss function.

29. The method of claim 28, wherein the calculating of the loss function comprises:
calculating a cross entropy loss function based on the third probability value; and
calculating a Kullback-Leibler divergence loss function based on the first probability value and the second probability value.

30. The method of claim 29, wherein the updating of the parameter includes summing the calculated cross entropy loss function and the calculated Kullback-Leibler divergence loss function and then performing backpropagation through an optimizer.

31. A computer comprising at least one processor that is implemented to execute a computer-readable instruction,
wherein the at least one processor trains a first artificial intelligence model that classifies an input image as osteoporosis or non-osteoporosis;
the at least one processor trains a second artificial intelligence model that classifies an input non-osteoporosis image as normal condition or osteopenia;
the at least one processor initially classifies a input image for diagnosis as the osteoporosis or the non-osteoporosis based on the first artificial intelligence model; and
the at least one processor classifies the initially classified non-osteoporosis image as the normal condition or the osteopenia based on the second artificial intelligence model.
